# FASCICULE DE BREVET EUROPEEN

(11) **EP 1 312 347 B1**
(45) Date de publication et mention de la délivrance du brevet: **02.02.2005**
(21) Numéro de dépôt: 02292780.0
(22) Date de dépôt: 07.11.2002
(51) Int. Cl.: A61K 7/13, C07H 15/203, C07H 17/00, C07D 498/04

(54) **Utilisation d'un extrait de myrsine africana en coloration d'oxydation pour la teinture des fibres kératiniques**
Verwendung eines Extrakts von Myrsine Afrikana für die oxidative Färbung von Keratin-Fasern
Use of an extract of myrsine africana for the oxidative dyeing of keratinic fibres

(30) Priorité: 14.11.2001 FR 0114719
(43) Date de publication de la demande: 21.05.2003
(73) Titulaire: L'OREAL, 75008 Paris (FR)
(72) Inventeur: Belcour-Castro, Béatrice, 94340 Joinville le Pont (FR); Burgaud, Hervé, 77230 Damartin en Goele (FR); Hussler, Georges, 93600 Aulnay sous Bois (FR); Seite, Michel, 75019 Paris (FR)
(74) Mandataire: Fevrier, Murielle

(56) Documents cités:
- EP-A- 0 776 652
- GB-A- 1 153 196

## Description

L'invention a pour objet l'utilisation d'un extrait de *Myrsine africana* en coloration d'oxydation pour la teinture de fibres kératiniques, de nouvelles compositions contenant cet extrait de plante, ainsi qu'un procédé de teinture par oxydation de fibres kératiniques mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il existe de plus un besoin constant de développer des compositions peu agressives pour la fibre kératinique, en particulier à partir de substance naturelle.

Par exemple, la demande de brevet EP 776 652 décrit l'utilisation d'extrait de plantes en coloration d'oxydation pour la teinture de fibres kératiniques. Les extraits de plantes mis en oeuvre dans cette demande sont des hydrolysats protéiniques.

Le but de la présente invention est de fournir de nouvelles compositions pour la teinture de fibres kératiniques par coloration d'oxydation à partir d'extrait de plantes qui respectent la nature de la fibre kératinique et présentent des teintures puissantes, peu sélectives et résistantes, capables d'engendrer de nouveaux colorants puissants pouvant donner des nuances variées.

Ce but est atteint avec la présente invention qui a pour objet l'utilisation d'un extrait de *Myrsine africana* comme précurseur de colorant pour la teinture de fibres kératiniques en coloration d'oxydation.

On appelle précurseur de colorant un composé non coloré qui, sous certaines conditions, se transforme en produit coloré.

L'invention a aussi pour objet une composition pour la teinture de fibres kératiniques par oxydation comprenant, dans un milieu approprié à la teinture des fibres kératiniques, un extrait de *Myrsine africana* et un composé de formule (I) ou l'un des sels correspondants d'acide ou de base cosmétiquement acceptables: 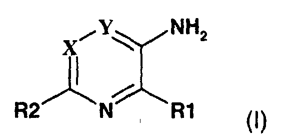 dans laquelle
- R₁ représente un radical NR₄R₅ dans lequel R₄ et R₅ représentent indépendamment l'un de l'autre un hydrogène ; un radical alkyle éventuellement substitué par un ou plusieurs groupes hydroxy, alcoxy, carboxy, amino, mono- ou di-alkylamino dont le radical alkyle est éventuellement substitué par un ou plusieurs groupes hydroxy, alcoxy, carboxy, amino, mono- ou di-alkylamino ; ou R₄ et R₅ forment ensemble avec l'atome d'azote auquel ils sont rattachés un hétérocycle saturé comprenant de 5 à 8 chaînons, l'hétérocycle pouvant être substitué par un ou plusieurs atomes d'halogène, un ou plusieurs radicaux hydroxy, alcoxy, carboxy, amino, carboxamido, sulfonamido, mono- ou di- alkylamino ou alkyle en C₁-C₆ éventuellement substitué par un ou plusieurs radicaux choisis parmi les radicaux hydroxy, amino, carboxy, mono- ou di- alkylamino, de préférence mono- ou di- alkylamino en C₁-C₂ ; l'hétérocycle pouvant contenir d'autres hétéroatomes choisis parmi O, S, SO₂ ou NR", R" représentant un atome d'hydrogène ou un radical alkyle en C₁-C₄,
- R₂ représente un radical NR₆R₇ dans lequel R₆ et R₇ représentent indépendamment l'un de l'autre un hydrogène ; un radical alkyle éventuellement substitué par un ou plusieurs groupes hydroxy, alcoxy, carboxy, amino, mono- ou di-alkylamino dont le radical alkyle est éventuellement substitué par un ou plusieurs groupes hydroxy, alcoxy, carboxy, amino, mono- ou di-alkylamino ; ou R₆ et R₇ forment ensemble avec l'atome d'azote auquel ils sont rattachés un hétérocycle saturé comprenant de 5 à 8 chaînons, l'hétérocycle pouvant être substitué par un ou plusieurs atomes d'halogènes, un ou plusieurs radicaux hydroxy, alcoxy, carboxy, amino, carboxamido, sulfonamido, mono- ou di- alkylamino ou alkyle en C₁-C₆ éventuellement substitué par un ou plusieurs radicaux choisis parmi les radicaux hydroxy, amino, carboxy, mono ou di alkylamino, de préférence mono- ou di- alkylamino en C₁-C₂ ; l'hétérocycle pouvant contenir d'autres hétéroatomes choisis parmi O, S, SO₂ ou NR", R" représentant un atome d'hydrogène ou un radical alkyle en C₁-C₄,
- X-Y représente RC-CR', N-CR avec R et R' choisis parmi un atome d'hydrogène, un radical alkyle, un radical hydroxyle ou un radical amino.

En effet, il a été découvert qu'à partir d'une composition comprenant un extrait de *Myrsine africana* et un composé de formule (I) ci dessus, on obtient une coloration par oxydation de fibres kératiniques, en particulier des cheveux dans des nuances nouvelles.

Dans le cadre de la présente invention, le terme alkyle signifie un radical saturé, linéaire ou ramifié comprenant, sauf autre indication, de 1 à 10 atomes de carbone, de préférence 1 à 6 atomes de carbone, et éventuellement substitué. Le terme alcoxy signifie Alkyl-O, le terme alkyle ayant la définition donnée ci dessus. Par amino, on entend le radical -NH₂. Par mono- ou di- alkylamino, on entend un radical amino dont un ou deux des atomes d'hydrogène sont remplacés par un radical alkyle tel que défini ci-dessus.

A titre d'exemple d'hétérocycle, on peut citer les cycles pyrrolidine, pipéridine, homopipéridine, pipérazine, homopipérazine et diazépane. Dans le cadre de l'invention, les hétérocycles ne contiennent pas de liaison peroxyde, ni de radicaux diazo ou nitroso.

Parmi les composés de formule (I), on peut citer à titre d'exemple, la 2,3-diamino 6-(pyrolidin 1-yl) pyridine, le 2,3-diamino 6-(4-hydroxypyrolidin 1-yl) pyridine, le 2-(pyrolidin 1-yl) 3-amino 6-(pyrolydin 1-yl) pyridine, la 2,4,5,6-tétra-aminopyrimidine, la 4-hydroxy 2,5,6-triaminopyrimidine, la 2,5,6-triaminopyrimidine.

Selon un premier mode de réalisation, le composé (I) correspond à la formule suivante : 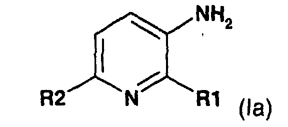 dans laquelle R₁ et R₂ sont tels que définis précédemment.

Selon un mode de réalisation particulier, R₁ représente NR₄R₅ dans lequel R₄ et R₅ représentent l'hydrogène, un radical alkyle, un radical hydroxyalkyle, ou R₄ et R₅ forment ensemble un cycle de 5 à 7 chaînons, R₂ représente NR₆R₇ dans laquelle R₆ et R₇ représentent l'hydrogène, un radical alkyle, un radical hydroxyalkyle, ou R₆ et R₇ forment ensemble un cycle de 5 à 7 chaînons.

En particulier, R₄ et R₅ ou R₆ et R₇ forment la 2,5-diméthylpyrrolidine, la proline, la 3-hydroxyproline, la 4-hydroxyproline, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthylpyrrolidine, la 3-hydroxypyrrolidine, la 3,4-dihydroxypyrrolidine, la 3-aminopyrrolidine, la 3-méthylaminopyrrolidine, la 4-amino-3-hydroxypyrrolidine, la 3-hydroxy-4-(2-hydroxyéthyl)aminopyrrolidine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, le diazépam, le N-méthyldiazépam, le N-(2-hydroxyéthyl)- diazépam.

De préférence, R₄ et R₅ représentent un hydrogène ou forment ensemble un cycle pyrrolidine. R₆ et R₇ forment de préférence un cycle pyrrolidin-1-yle, pouvant être substitué.

Selon ce premier mode de réalisation, les composés (I) correspondant à l'une des formules suivantes sont préférés : 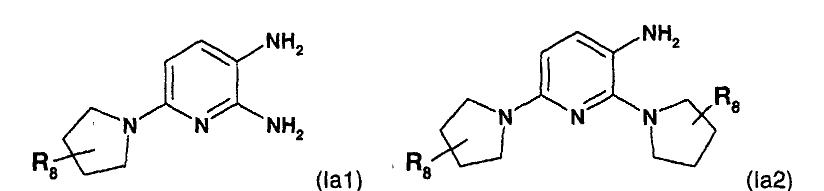 dans laquelle R₈ représente un atome d'hydrogène, un atome d'halogène, un radical hydroxy, alcoxy, carboxy, amino, carboxamido, sulfonamido, mono- ou di- alkylamino ou un radical alkyle en C₁-C₆ éventuellement substitué par un ou plusieurs radicaux hydroxy, amino, carboxy, mono- ou di- alkylamino, de préférence mono- ou di-alkylamino en C₁-C₂.

Les composés (I) particulièrement préférés sont choisis parmi la 2,3-diamino 6-(pyrrolidin 1-yl)pyridine, la 2,3-diamino-6-(4-hydroxypyrrolidin 1-yl) pyridine, la 2-(pyrrolidin-1-yl)-3-amino 6-(pyrrolydin 1-yl)pyridine.

Selon un deuxième mode de réalisation, le composé (I) correspond à la formule suivante : 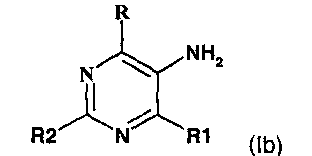 dans laquelle R₁, R₂ et R sont tels que définis précédemment.

Selon un mode de réalisation particulier, R est un radical amino ou hydroxy. De façon préférée, R, R₁ et R₂ représentent NH₂.

*Myrsine africana* est une plante de la famille des *Myrsinaceae,* dénommée Cape myrtle ou African Boxwood en langue vernaculaire et que l'on trouve en Afrique, mais également dans l'ouest de la Chine et dans l'Himalaya. C'est un arbuste, à feuilles pérennes, à croissance très lente et à fleurs monoïques, dont le fruit est à simple graine.

L'extrait de *Myrsine africana* peut être obtenu selon les techniques classiques d'extraction de plantes. Toute méthode d'extraction connue de l'homme du métier peut être utilisée pour préparer cet extrait. On peut, en particulier, citer les extraits alcooliques, notamment éthanoliques ou encore hydroalcooliques.

De telles techniques sont par exemple décrites dans "Methods in Plant Biochemistry", vol1, 1989, Dey and Harborne, Academic Press edition . Ces extraits peuvent être obtenus à partir de la plante entière, la plante pouvant être à différents stades de maturité physiologique. Il est aussi possible d'utiliser des souches cellulaires issues de cette plante. Les souches cellulaires peuvent être obtenues selon des techniques décrites dans " Plant tissue culture : théorie and practice" -1953, Bhojwani et Razdan, édition Elviser ou " La culture des tissus végétaux"- 1959, Gautheret, édition Masson.

On peut également utiliser un extrait de *Myrsine africana* préparé par la méthode décrite dans FR 2 731 162 (la demande de brevet français n° 95-02379 déposée par la demanderesse). Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.

D'autre part, la première étape peut avantageusement être remplacée par une opération de congélation simple des tissus végétaux (par exemple à -20°C), suivie d'une extraction aqueuse reprenant les deuxième et troisième étapes ci-dessus décrites. Le produit de cette extraction aqueuse peut également être lyophilisé pour obtenir un extrait sec (en moyenne selon la nature de la source initiale, on retrouve entre 5 et 70 g de matière sèche par litre d'extrait aqueux).

Selon un mode de réalisation particulier, l'extrait de plante est un extrait aqueux obtenu à partir de la partie aérienne de la plante (tiges et feuilles) de *Myrsine africana*.

L'extrait de *Myrsine africana* contient en particulier le 3-(β-D-Glucopyranosyloxy)-4,5-dihydroxytoluène, composé de formule (II) ci-dessous. L'invention a aussi pour objet le composé (II) susceptible d'être obtenu à partir d'un extrait de *Myrsine africana .*

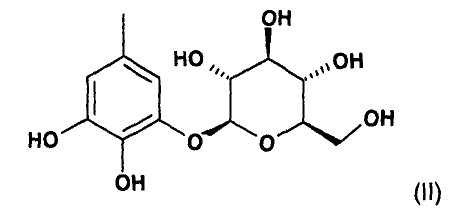

Un autre objet de l'invention est une composition pour la teinture par oxydation de fibres kératiniques comprenant, dans un milieu approprié pour la teinture des fibres kératiniques, un précurseur de colorant de formule (I) tel que défini précédemment et du 3-(β-D-Glucopyranosyloxy)-4,5-dihydroxytoluène susceptible d'être obtenu à partir d'un extrait aqueux de *Myrsine africana.*

Par oxydation de la composition selon l'invention, il se forme dans le milieu un produit coloré résultant de la condensation oxydative du composé (I) avec le composé (II). A titre d'exemple, à partir d'une composition de l'invention qui contient le 3-(β-D-Glucopyranosyloxy)-4,5-dihydroxytoluène et un composé (la1) suivant, 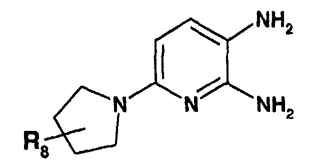 il se forme les produits colorés (IIIa), (IIIb) et (IIIc) décrits ci après : 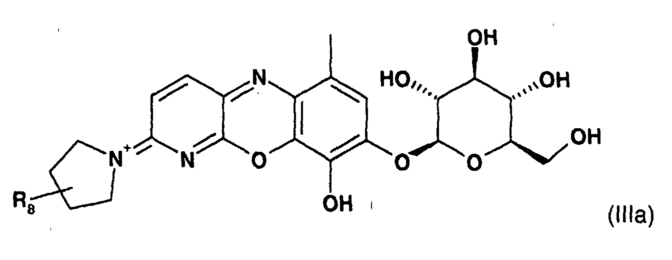 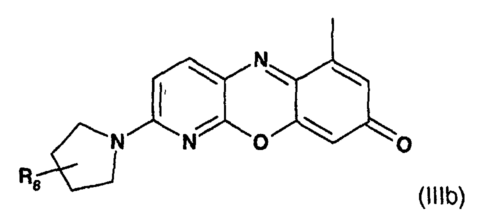 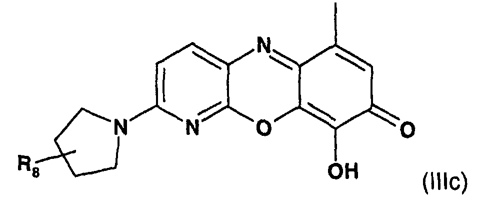 R₈ étant tel que défini précédemment.

Ces trois produits colorés résultent d'un couplage oxydatif du composé diaminopyridine et du dérivé glucosylé de trihydroxytoluène, couplage suivi d'une substitution nucléophile du groupement amino en C-2 du composé diaminopyridine par un groupement OH du dérivé trihydroxytoluène, cette cyclisation entraînant la perte d'ammoniac. D'autres produits colorés peuvent être obtenus selon le même mécanisme à partir des composés de formule (I).

L'invention a ainsi pour autre objet les produits colorés susceptibles d'être obtenus à partir de la composition selon la présente invention. Ces produits colorés peuvent notamment se présenter sous la forme de pigments et être utilisés à titre de colorant direct pour la teinture directe des cheveux ou bien encore être incorporés dans des produits cosmétiques tels que par exemple dans des produits de maquillage.

On a de plus observé que l'autocondensation oxydative des composés de formule (I), en particulier (Ia1) et (Ia2) est favorisée en présence d'extrait de *Myrsine africana.* En effet, l'apparition de la couleur est plus rapide en présence de *Myrsine africana*.

Dans la composition de la présente invention, l'extrait de *Myrsine africana* est présent en quantité comprise entre 0,1% et 10% en poids par rapport au poids de la composition, de préférence entre 0,5 et 5 %.

Dans le cadre de la présente invention, la composition peut de plus contenir une ou plusieurs bases d'oxydation classiques en teinture d'oxydation, par exemple les paraphénylènediamines, les bis-phénylalkylènediamines, les paraaminophénols, les orthoaminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloroparaphénylènediamine, la 2,3-diméthylparaphénylènediamine, la 2,6-diméthylparaphénylènediamine, la 2,6-diéthylparaphénylènediamine, la 2,5-diméthylparaphénylènediamine, la N,N-diméthylparaphénylènediamine, la N,N-diéthylparaphénylènediamine, la N,N-diéthylparaphénylènediamine, la N,N-dipropylparaphénylènediamine, la 4-amino-N,N-diéthyl-3-méthylaniline, la N,N-bis-(β-hydroxyéthyl)paraphénylènediamine, la 4-N,N-bis-(β-hydroxyéthyl)amino-2-méthylaniline, la 4-N,N-bis-(β-hydroxyéthyl)amino-2-chloroaniline, la 2-β-hydroxyéthylparaphénylènediamine, la 2-fluoroparaphénylènediamine, la 2-isopropylparaphénylènediamine, la N-(β-hydroxypropyl)paraphénylènediamine, la 2-hydroxyméthylparaphénylènediamine, la N,N-diméthyl-3-méthyl paraphénylènediamine, la N,N-(éthyl, β-hydroxyéthyl)paraphénylènediamine, la N-(β,γ-dihydroxypropyl)paraphénylènediamine, la N-(4'-aminophényl) paraphénylènediamine, la N-phénylparaphénylènediamine, la 2-β-hydroxyéthyloxy paraphénylènediamine, la 2-β-acétylaminoéthyloxyparaphénylènediamine, la N-(β-méthoxyéthyl)paraphénylènediamine, la 4-aminophénylpyrrolidine, la 2 thiénylparaphénylènediamine, le 2-β-hydroxyéthylamino-5-aminotoluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2-β-hydroxyéthylparaphénylènediamine, la 2-β-hydroxyéthyloxyparaphénylènediamine, la 2,6-diméthylparaphénylènediamine, la 2,6-diéthylparaphénylènediamine, la 2,3-diméthyl paraphénylènediamine, la N,N-bis-(β-hydroxyéthyl)paraphénylènediamine, la 2-chloro paraphénylènediamine, la 2-β-acétylaminoéthyloxyparaphénylènediamine, et leurs sels d'addition avec un acide sont particulièrement préférés.

Parmi les bis-phénylalkylènediamines, on peut citer à titre d'exemple, le N,N'-bis-(β-hydroxyéthyl)-N,N'-bis-(4'-aminophényl)-1,3-diaminopropanol, la N,N'-bis-(β-hydroxyéthyl)-N,N'-bis-(4'-aminophényl)éthylènediamine, la N,N'-bis-(4-aminophényl)tétraméthylènediamine, la N,N'-bis-(β-hydroxyéthyl)-N,N'-bis-(4-aminophényl)tétraméthylènediamine, la N,N'-bis-(4-méthylaminophényl)tétraméthylènediamine, la N,N'-bis-(éthyl)-N,N'-bis-(4'-amino, 3'-méthylphényl)éthylènediamine, le 1,8-bis-(2,5-diaminophénoxy)-3,6-dioxaoctane, et leurs sels d'addition avec un acide.

Parmi les paraaminophénols, on peut citer à titre d'exemple, le paraaminophénol, le 4-amino-3-méthylphénol, le 4-amino-3-fluorophénol, le 4-amino-3-hydroxyméthylphénol, le 4-amino-2-méthylphénol, le 4-amino-2-hydroxyméthylphénol, le 4-amino-2-méthoxyméthylphénol, le 4-amino-2-aminométhylphénol, le 4-amino-2-(β-hydroxyéthylaminométhyl)phénol, le 4-amino-2-fluorophénol, et leurs sels d'addition avec un acide.

Parmi les orthoaminophénols, on peut citer à titre d'exemple, le 2-aminophénol, le 2-amino-5-méthylphénol, le 2-amino-6-méthylphénol, le 5-acétamido-2-aminophénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diaminopyridine, la 2-(4-méthoxyphényl)amino-3-aminopyridine, la 3,4-diaminopyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2-hydroxy-4,5,6-triaminopyrimidine, la 2,4-dihydroxy-5,6-diaminopyrimidine, et les dérivés pyrazolopyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthylpyrazolo-[1,5-a]-pyrimidine-3,7-diamine ; la pyrazolo-[1,5-a]-pyrimidine-3,5-diamine ; la 2,7-diméthylpyrazolo-[1,5-a]-pyrimidine-3,5-diamine ; le 3-amino-pyrazolo[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol, le 2-(7-amino-pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthylpyrazolo-[1,5-a]-pyrimidine-3,7-diamine, la 2, 5, N 7, N 7-tetraméthyl-pyrazolo-[1,5-a]-pyrimidine-3,7-diamine, la 3-amino-5-méthyl-7-imidazolylpropylamino-pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthylpyrazole, le 4,5-diamino-1-(β-hydroxyéthyl)pyrazole, le 3,4-diaminopyrazole, le 4,5-diamino-1-(4'-chlorobenzyl)pyrazole, le 4,5-diamino-1,3-diméthylpyrazole, le 4,5-diamino-3-méthyl-1-phénylpyrazole, le 4,5-diamino-1-méthyl-3-phénylpyrazole, le 4-amino-1,3-diméthyl-5-hydrazino pyrazole, le 1-benzyl-4,5-diamino-3-méthylpyrazole, le 4,5-diamino-3-tert-butyl-1-méthyl pyrazole, le 4,5-diamino-1-tert-butyl-3-méthylpyrazole, le 4,5-diamino-1-(β-hydroxyéthyl)-3-méthylpyrazole, le 4,5-diamino-1-éthyl-3-méthylpyrazole, le 4,5-diamino-1-éthyl-3-(4'-méthoxyphényl)pyrazole, le 4,5-diamino-1-éthyl 3-hydroxyméthylpyrazole, le 4,5-diamino-3-hydroxyméthyl-1-méthylpyrazole, le 4,5-diamino-3-hydroxyméthyl-1-isopropylpyrazole, le 4,5-diamino-3-méthyl 1-isopropylpyrazole, le 4-amino-5-(2'-aminoéthyl)amino-1,3-diméthyl pyrazole, le 3,4,5-triaminopyrazole, le 1-méthyl-3,4,5-triaminopyrazole, le 3,5-diamino-1-méthyl-4-méthylaminopyrazole, le 3,5-diamino-4-(β-hydroxyéthyl)amino-1-méthylpyrazole, et leurs sels d'addition avec un acide.

La composition selon l'invention peut contenir un ou plusieurs coupleurs qui sont conventionnellement utilisés pour la teinture de fibres kératiniques. Parmi ces coupleurs, on peut notamment citer les métaphénylènediamines, les métaaminophénols, les métadiphénols, les coupleurs naphtaléniques et les coupleurs hétérocycliques ainsi que leur sels d'addition.

A titre d'exemple, on peut citer le 2-méthyl-5-aminophénol, le 5-N-(β-hydroxyéthyl)amino-2-méthylphénol, le 6-chloro-2-méthyl-5-aminophénol, le 3-aminophénol, le 1,3-dihydroxybenzène, le 1,3-dihydroxy-2-méthylbenzène, le 4-chloro-1,3-dihydroxybenzène, le 2,4-diamino-1-(β-hydroxyéthyloxy)benzène, le 2-amino,-4-(β-hydroxyéthylamino)-1-méthoxybenzène, le 1,3-diaminobenzène, le 1,3-bis-(2,4-diaminophénoxy)propane, la 3-uréidoaniline, le 3-uréido-1-diméthylaminobenzène, le sésamoi, le 1-β-hydroxyéthylamino-3,4-méthylènedioxybenzène, l'α-naphtol, le 2-méthyl-1-naphtol, le 6-hydroxyindole, le 4-hydroxyindole, le 4-hydroxy-N-méthylindole, la 2-amino-3-hydroxypyridine, la 6-hydroxybenzomorpholine, la 3,5-diamino-2,6-diméthoxypyridine, le 1-N-(β-hydroxyéthyl)amino-3,4-méthylènedioxybenzène, le 2,6-bis-(β-hydroxyéthylamino)toluène et leurs sels d'addition.

Dans la composition de la présente invention, les bases d'oxydations et les coupleurs additionnels sont en général présents en quantité de comprise entre 0,001 et 10 % en poids environ du poids total de la composition tinctoriale et plus préférentiellement de 0,005 à 6 %.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition avec une base utilisables dans le cadre de l'invention sont par exemple choisis parmi les sels d'addition avec la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs cationiques, les colorants directs azoïques, méthiniques, azométhiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 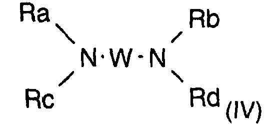 dans laquelle W est un reste propylène éventuellement substitué par un groupement hydroxyle ou un radical alkyle en C1-C4 ; Ra, Rb, Rc et Rd, identiques ou différents, représentent un atome d'hydrogène, un radical alkyle en C1-C4 ou hydroxyalkyle en C1-C4.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux caractérisé en ce qu'on applique sur les fibres la composition de la présente invention telle que définie précédemment, et qu'on révèle la couleur par l'oxygène de l'air ou à l'aide d'un agent oxydant.

La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées. Selon un mode de réalisation différent, l'agent oxydant est appliqué sur les fibres sous forme de composition oxydante simultanément ou séquentiellement à la composition de l'invention.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

### Préparation de l'extrait de Myrsine africana

L'extrait utilisé est un extrait aqueux de partie aérienne de *Myrsine africana* brut et /ou fractionné. Par partie aérienne, on entend la partie hors sol de la plante, en l'occurrence, les tiges et les feuilles. La plante n'était pas en floraison lors de la réalisation de cet extrait.,

L'ensemble feuilles et tiges est prélevé, séché à 45°C, broyé sur broyeur à couteaux, puis tamisé sur un tamis de maille égale à 0,5 µm.

La poudre ainsi obtenue est extraite par agitation en présence d'eau carbonatée à pH 9,5 préparée comme suit : du carbonate anhydre de sodium est ajouté à 1g/l à de l'eau distillée, le pH est ajusté à 9,5 par addition d'HCl 1N.

L'extraction est réalisée à température ambiante, à raison de 5 g de poudre de plante pour 100 ml d'eau carbonatée, placé en agitation 1h30 à 900 tours par minute.

Le mélange est ensuite filtré sous vide sur filtre de 2,7 µm de porosité. Le filtrat obtenu est ensuite congelé puis lyophilisé.

### Préparation des compositions tinctoriales

### 1. Coloration en solution

**1.1 -** Les composés (IA) ou (IB) qui sont décrits ci après sont mis en solution dans un tampon à pH 9,5 à une concentration de 1%. L'extrait de *Myrsine africana* préparé selon le mode opératoire ci dessus est mis en solution dans de l'eau à une concentration de 1%. 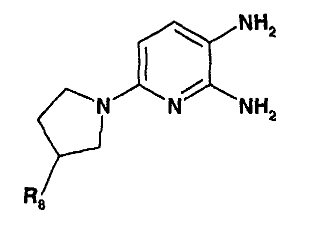 (IA) avec R₈ = H
   (IB) avec R₈ = OH
   A un mélange de ces deux solutions dans un rapport pondéral 1/1, on ajoute de l'eau oxygénée à 3%.
   Après 10 minutes à température ambiante, on observe l'apparition d'une coloration rouge violacée.
   En réalisant la même expérience en l'absence de *Myrsine africana,* on obtient une coloration verte.
   La même opération est réalisée après mise en solution des composés (IA) ou (IB) décrits ci-dessous dans une solution tampon à pH 7, la coloration obtenue est alors bleu-vert. En réalisant la même expérience en l'absence de *Myrsine africana,* on obtient une coloration verte.
   Des exemples ont été réalisés à partir des solutions de composés (IA) ou (IB) décrits ci dessus à pH 7 et pH 9,5 et d'une solution d'extrait de *Myrsine africana* préparée selon le mode opératoire ci dessus mais sans ajout d'eau oxygénée. Les mêmes colorations sont observées par oxydation avec l'oxygène de l'air.
**1.2 -** Un exemple a été réalisé selon le mode opératoire ci dessus à partir d'une composition contenant un extrait de *Myrsine africana* et 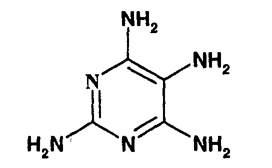
   On obtient à pH 9,5 une coloration orangée et à pH 7 une coloration rosée. En l'absence de Myrsine africana, la coloration est jaune.
**1.3 -** Un exemple a été réalisé selon le mode opératoire ci dessus à partir d'une composition contenant un extrait de *Myrsine africana* et 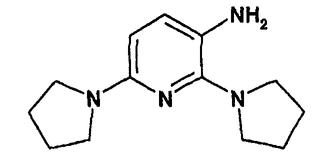
   On obtient à pH 9,5 une coloration rouge violacée et à pH 7 une coloration bleu-vert.

### 2. Coloration de fibres kératiniques

Les expériences ci dessus ont été réalisées en mettant en contact la solution résultant du mélange de la solution de précurseurs de colorant et d'extrait de *Myrsine africana* avec des poils de chèvre selon le mode opératoire suivant : Les poils de chèvre sont mis en contact avec 1 ml d'extrait aqueux de *Myrsine africana* à 1 %, puis est ajouté 1 ml de la solution de composés (IA) ou (IB) à 1 % dans le tampon pH 9.5. Après 20 minutes, les poils de chèvre sont retirés du mélange, rincés à l'eau et séchés à température ambiante. Ils apparaissent teintés en rouge violacé. La même expérience a été réalisée en présence d'eau oxygénée. On obtient ainsi la même coloration rouge violacée.

La même réaction est réalisée à pH 7 et a permis de teindre le poil de chèvre en bleu vert.

## Revendications

1. Utilisation d'un extrait de *Myrsine africana* comme précurseur de colorant pour la teinture de fibres kératiniques en coloration d'oxydation.

2. Composition pour la teinture de fibres kératiniques par oxydation comprenant, dans un milieu approprié à la teinture des fibres kératiniques, un extrait de *Myrsine africana* et un composé de formule (I) ou l'un des sels correspondants d'acide ou de base cosmétiquement acceptables: 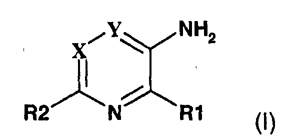 dans laquelle
• R₁ représente un radical NR₄R₅ dans lequel R₄ et R₅ représentent indépendamment l'un de l'autre un hydrogène ; un radical alkyle éventuellement substitué par un ou plusieurs groupes hydroxy, alcoxy, carboxy, amino, mono- ou dialkylamino dont le radical alkyle est éventuellement substitué par un ou plusieurs groupes hydroxy, alcoxy, carboxy, amino, mono- ou di-alkylamino ; ou R₄ et R₅ forment ensemble avec l'atome d'azote auquel ils sont rattachés un hétérocycle saturé comprenant de 5 à 8 chaînons, l'hétérocycle pouvant être substitué par un ou plusieurs atomes d'halogène, un ou plusieurs radicaux hydroxy, alcoxy, carboxy, amino, carboxamido, sulfonamido, mono- ou di- alkylamino ou alkyle en C₁-C₆ éventuellement substitué par un ou plusieurs radicaux choisis parmi les radicaux hydroxy, amino, carboxy, mono- ou di- alkylamino, de préférence mono- ou di- alkylamino en C₁-C₂ ; l'hétérocycle pouvant contenir d'autres hétéroatomes choisis parmi O, S, SO2 ou NR", R" représentant un atome d'hydrogène ou un radical alkyle en C₁-C₄,
• R₂ représente un radical NR₆R₇ dans lequel R₆ et R₇ représentent indépendamment l'un de l'autre un hydrogène ; un radical alkyle éventuellement substitué par un ou plusieurs groupes hydroxy, alcoxy, carboxy, amino, mono- ou dialkylamino dont le radical alkyle est éventuellement substitué par un ou plusieurs groupes hydroxy, alcoxy, carboxy, amino, mono- ou di-alkylamino ; ou R₆ et R₇ forment ensemble avec l'atome d'azote auquel ils sont rattachés un hétérocycle saturé comprenant de 5 à 8 chaînons, l'hétérocycle pouvant être substitué par un ou plusieurs atomes d'halogènes, un ou plusieurs radicaux hydroxy, alcoxy, carboxy, amino, carboxamido, sulfonamido, mono- ou di- alkylamino ou alkyle en C₁-C₆ éventuellement substitué par un ou plusieurs radicaux choisis parmi les radicaux hydroxy, amino, carboxy, mono ou di alkylamino, de préférence mono- ou di- alkylamino en C₁-C₂ ; l'hétérocycle pouvant contenir d'autres hétéroatomes choisis parmi O, S, SO₂ ou NR", R" représentant un atome d'hydrogène ou un radical alkyle en C₁-C₄,
• X-Y représente RC-CR', N-CR avec R et R' choisis parmi un atome d'hydrogène, un radical alkyle, un radical hydroxyle ou un radical amino.

3. Composition selon la revendication 2 dans laquelle le composé (I) correspond à la formule suivante : 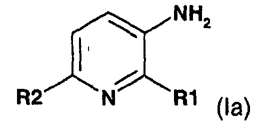 dans laquelle R₁ et R₂ sont tels que définis à la revendication 2.

4. Composition selon la revendication 3 dans laquelle R₁ représente NR₄R₅ dans laquelle R₄ et R₅ représentent l'hydrogène, un radical alkyle, un radical hydroxyalkyle, ou R₄ et R₅ forment ensemble un cycle de 5 à 7 chaînons, R₂ représente NR₆R₇ dans laquelle R₆ et R₇ représentent l'hydrogène, un radical alkyle, un radical hydroxyalkyle, ou R₆ et R₇ forment ensemble un cycle de 5 à 7 chaînons.

5. Composition selon la revendication 3 ou 4 dans laquelle , R₄ et R₅ représentent un hydrogène ou forment ensemble un cycle pyrrolidine, R₆ et R₇ forment de préférence un cycle pyrrolidin 1-yle, pouvant être substitué.

6. Composition selon l'une quelconque des revendications 2 à 5 dans laquelle le composé (I) correspond à l'une des formules suivantes 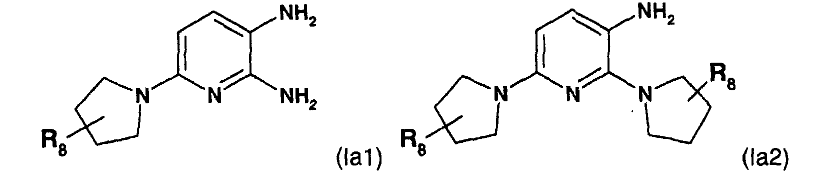 dans laquelle R₈ représente un atome d'hydrogène, un atome d'halogène, un radical hydroxy, alcoxy, carboxy, amino, carboxamido, sulfonamido, mono- ou di- alkylamino ou un radical alkyle en C₁-C₆ éventuellement substitué par un ou plusieurs radicaux hydroxy, amino, carboxy, mono- ou di- alkylamino, de préférence mono- ou di- alkylamino en C₁-C_{2.}

7. Composition selon la revendication 2 dans laquelle le composé (I) correspond à la formule suivante : 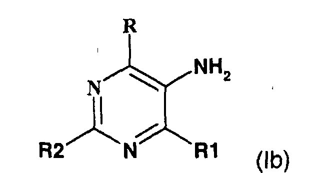 dans laquelle R, R₁ et R₂ sont tels que définis dans la revendication 2.

8. Composition selon la revendication 7 dans laquelle le composé (Ib) est tel que R est un radical amino ou hydroxy.

9. Composition selon la revendication 8 dans laquelle le composé (Ib) est tel que R, R₁ et R₂ représentent NH₂.

10. Composition selon l'une quelconque des revendications 2 à 9 comprenant un composé de formule (II) suivante

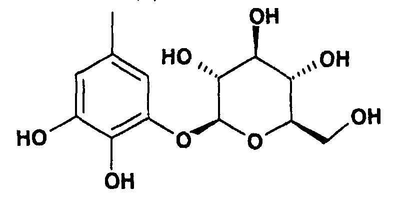

11. Composition selon l'une quelconque des revendications 2 à 10 dans laquelle la quantité d'extrait de *Myrsine africana* est comprise entre 0,1% et 10% en poids.

12. Composition selon l'une quelconque des revendications 2 à 11, comprenant de plus une ou plusieurs bases d'oxydation sont choisies parmi les paraphénylènediamines, les bis-phénylalkylènediamines, les para-aminophénols, les ortho-aminophénols, les bases hétérocycliques, et leurs sels d'addition.

13. Composition selon l'une quelconque des revendications 2 à 12, comprenant de plus un ou plusieurs coupleurs choisis parmi les métaphénylènediamines, les méta-aminophénols, les métadiphénols, les coupleurs naphtaléniques et les coupleurs hétérocycliques ainsi que leur sels d'addition.

14. Composition selon l'une quelconque des revendications 2 à 13 comprenant de plus un colorant direct.

15. Composition pour la teinture par oxydation de fibres kératiniques comprenant, dans un milieu approprié pour la teinture des fibres kératiniques, un précurseur de colorant de formule (I) tel que défini dans l'une quelconque des revendications 2 à 9 et du 3-(β-D-Glucopyranosyloxy)-4,5-dihydroxytoluène susceptible d'être obtenu à partir d'un extrait aqueux de *Myrsine africana*

16. Procédé de teinture d'oxydation des fibres kératiniques et en particulier des fibres kératiniques humaines telles que les cheveux, **caractérisé en ce qu'**on applique sur les fibres au moins une composition telle que définie à l'une quelconque des revendications 2 à 15, et qu'on révèle la couleur par l'oxygène de l'air ou à l'aide d'un agent oxydant.

17. Procédé selon la revendication 16 dans lequel l'agent oxydant est choisi parmi le peroxyde d'hydrogène, le peroxyde d'urée, les bromates de métaux alcalins, les persels, les peracides et les enzymes oxydases.

18. Procédé selon l'une des revendications 16 ou 17 dans lequel l'agent oxydant est mélangé au moment de l'emploi à la composition.

19. Procédé selon l'une quelconque des revendications 17 ou 18 dans lequel l'agent oxydant est appliqué sur les fibres sous forme de composition oxydante simultanément ou séquentiellement à la composition telle que définie selon l'une quelconque des revendications 2 à 15.

20. Dispositif à plusieurs compartiments ou "kit" de teinture à plusieurs compartiments, dans lequel un premier compartiment contient une composition telle que définie à l'une quelconque des revendications 2 à 15 et un deuxième compartiment contient une composition oxydante.

21. Produit coloré susceptible d'être obtenu à partir de la composition telle que définie à la revendication 14 par réaction du composé (I) avec le composé (II).

22. Produit selon la revendication 21 choisi parmi les produits de formules (IIIa), (IIIb) et (IIIc) et leurs mélanges : 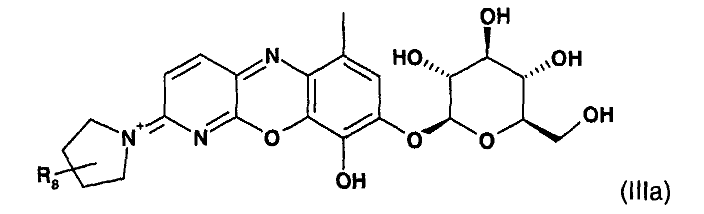 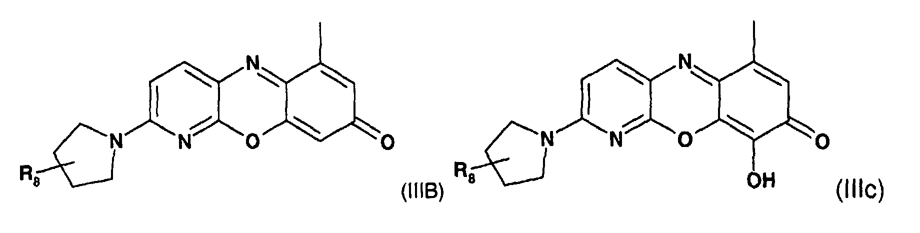 dans lesquelles R₈ est tel que défini à la revendication 6.

23. Composé de formule suivante susceptible d'être obtenu à partir d'un extrait de *Myrsine africana* :

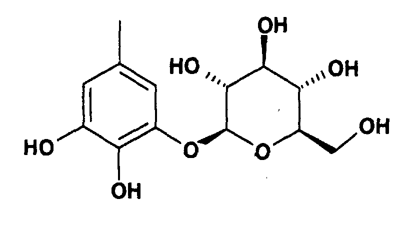

## Patentansprüche

1. Verwendung eines Extrakts von *Myrsine africana* als Farbstoffvorprodukt eines Oxidationsfarbstoffes zum oxidativen Färben von Keratinfasern.

2. Zusammensetzung zum oxidativen Färben von Keratinfasern, die in einem zum Färben von Keratinfasern geeigneten Medium einen Extrakt von *Myrsine africana* und eine Verbindung der Formel (I) oder ein Salz einer Verbindung der Formel (I) mit einer kosmetisch akzeptablen Säure oder Base enthält: 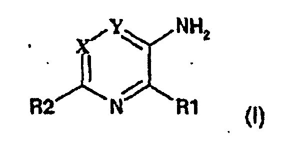 worin bedeuten:
- R₁ eine Gruppe NR₄R₅, worin R₄ und R₅ unabhängig voneinander Wasserstoff; oder eine Alkylgruppe bedeuten, die gegebenenfalls mit einer oder mehreren der folgenden Gruppen substituiert ist: Hydroxy, Alkoxy, Carboxy, Amino, Mono- oder Dialkylamino, deren Alkylgruppe gegebenenfalls mit einer oder mehreren Hydroxygruppen, Alkoxygruppen, Carboxygruppen, Aminogruppen, Monoalkylaminogruppen oder Dialkylaminogruppen substituiert ist; oder R₄ und R₅ bilden gemeinsam mit dem Stickstoffatom, an das sie gebunden sind, einen gesättigten, 5- bis 8-gliedrigen Heterocyclus, wobei der Heterocyclus mit einem oder mehreren Halogenatomen, einer oder mehreren Gruppen Hydroxy, Alkoxy, Carboxy, Amino, Carboxamido, Sulfonamido, Mono- oder Dialkylamino oder einer Alkylgruppe mit 1 bis 6 Kohlenstoffatomen substituiert sein kann, die gegebenenfalls mit einer oder mehreren Gruppen substituiert ist, die unter den Gruppen Hydroxy, Amino, Carboxy, Mono- oder Dialkylamino und vorzugsweise Mono- oder Dialkylamino mit 1 bis 2 Kohlenstoffatomen ausgewählt sind; wobei der Heterocyclus weitere Heteroatome enthalten kann, die unter O, S, SO₂ oder NR" ausgewählt sind, wobei R" ein Wasserstoffatom oder eine C₁₋₄-Alkylgruppe bedeutet.
- R₂ eine Gruppe NR₆R₇, worin R₆ und R₇ unabhängig voneinander Wasserstoff; oder eine Alkylgruppe bedeuten, die gegebenenfalls mit einer oder mehreren der folgenden Gruppen substituiert ist: Hydroxy, Alkoxy, Carboxy, Amino, Mono- oder Dialkylamino, deren Alkylgruppe gegebenenfalls mit einer oder mehreren Hydroxygruppen, Alkoxygruppen, Carboxygruppen, Aminogruppen, Monoalkylaminogruppen oder Dialkylaminogruppen substituiert ist; oder R₆ und R₇ bilden gemeinsam mit dem Stickstoffatom, an das sie gebunden sind, einen gesättigten, 5- bis 8-gliedrigen Heterocyclus, wobei der Heterocyclus mit einem oder mehreren Halogenatomen, einer oder mehreren Gruppen Hydroxy, Alkoxy, Carboxy, Amino, Carboxamido, Sulfonamido, Mono- oder Dialkylamino oder einer Alkylgruppe mit 1 bis 6 Kohlenstoffatomen substituiert sein kann, die gegebenenfalls mit einer oder mehreren Gruppen substituiert ist, die unter den Gruppen Hydroxy, Amino, Carboxy, Mono- oder Dialkylamino und vorzugsweise Mono- oder Dialkylamino mit 1 bis 2 Kohlenstoffatomen ausgewählt sind; wobei der Heterocyclus weitere Heteroatome enthalten kann, die unter O, S, SO₂ oder NR" ausgewählt sind, wobei R" ein Wasserstoffatom oder eine C₁₋₄-Alkylgruppe bedeutet,
- X-Y RC-CR', N-CR, wobei R und R' unter Wasserstoff, Alkyl, Hydroxy oder Amino ausgewählt sind.

3. Zusammensetzung nach Anspruch 2, wobei die Verbindung (I) der folgenden Formel entspricht: 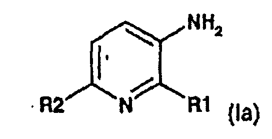 worin R₁ und R₂ die in Anspruch 2 angegebenen Bedeutungen aufweisen.

4. Zusammensetzung nach Anspruch 3, wobei R₁ NR₄R₅ bedeutet, worin R₄ und R₅ Wasserstoff, Alkyl oder Hydroxyalkyl bedeuten oder R₄ und R₅ gemeinsam einen 5- bis 7-gliedrigen Ring bilden, und R₂ NR₆R₇ bedeutet, worin R₆ und R₇ Wasserstoff, Alkyl oder Hydroxyalkyl bedeuten oder R₆ und R₇ gemeinsam einen 5- bis 7-gliedrigen Ring bilden.

5. Zusammensetzung nach Anspruch 3 oder 4, worin R₄ und R₅ ein Wasserstoffatom bedeuten oder gemeinsam einen Pyrrolidinring bilden und R₆ und R₇ vorzugsweise einen Pyrrolidin-1-yl-Ring bilden, der substituiert sein kann.

6. Zusammensetzung nach einem der Ansprüche 2 bis 5, wobei die Verbindung (I) einer der folgenden Formeln entspricht 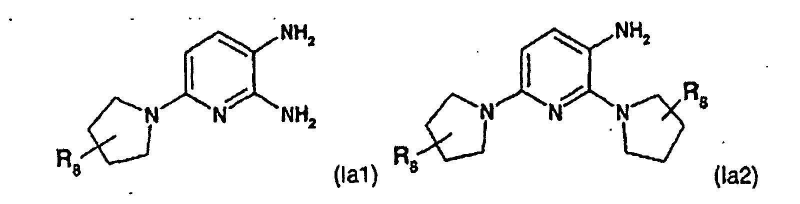 worin R₈ ein Wasserstoffatom, ein Halogenatom, Hydroxy, Alkoxy, Carboxy, Amino, Carboxamido, Sulfonamido, Mono- oder Dialkylamino oder eine C₁₋₆-Alkylgruppe bedeutet, die gegebenenfalls mit einer oder mehreren Gruppen Hydroxy, Amino, Carboxy, Mono- oder Dialkylamino und vorzugsweise Mono- oder Dialkylamino mit 1 bis 2 Kohlenstoffatomen substituiert ist.

7. Zusammensetzung nach Anspruch 2, wobei die Verbindung (I) der folgenden Formel entspricht: 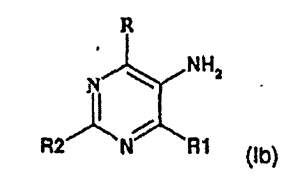 worin R, R₁ und R₂ die in Anspruch 2 angegebenen Bedeutungen aufweisen.

8. Zusammensetzung nach Anspruch 7, wobei in der Verbindung (Ib) R eine Aminogruppe oder eine Hydroxygruppe bedeutet.

9. Zusammensetzung nach Anspruch 8, wobei in der Verbindung (Ib) R, R₁ und R₂ NH₂ bedeuten.

10. Zusammensetzung nach einem der Ansprüche 2 bis 9, die eine Verbindung der folgenden Formel (II) enthält

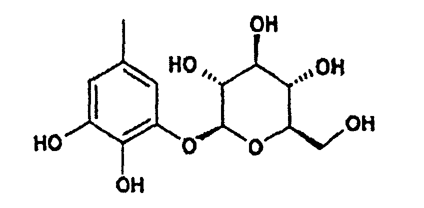

11. Zusammensetzung nach einem der Ansprüche 2 bis 10, wobei die Menge des Extraktes von *Myrsine africana* im Bereich von 0, 1 bis 10 Gew.-% liegt.

12. Zusammensetzung nach einem der Ansprüche 2 bis 11, die eine oder mehrere Oxidationsbasen enthält, die unter den *p*-Phenylendiaminen, Bisphenylalkylendiaminen, *p*-Aminophenolen, *o*-Aminophenolen, heterocyclischen Basen und deren Additionssalzen ausgewählt sind.

13. Zusammensetzung nach einem der Ansprüche 2 bis 12, die einen oder mehrere Kuppler enthält, die unter den *m*-Phenylendiaminen, *m*-Aminophenolen, *m*-Dihydroxybenzolen, Naphthalinkupplern und heterocyclischen Kupplern sowie deren Additionssalzen ausgewählt sind.

14. Zusammensetzung nach einem der Ansprüche 2 bis 13, die ferner einen Direktfarbstoff enthält.

15. Zusammensetzung zum oxidativen Färben von Keratinfasern, die in einem zum Färben von Keratinfasern geeigneten Medium ein Farbstoffvorprodukt eines Oxidationsfarbstoffes der Formel (I) nach einem der Ansprüche 2 bis 9 und das 3-(β-D-Glucopyranosyloxy)-4,5-dihydroxytoluol enthält, das aus einem wässerigen Extrakt von *Myrsine africana* erhältlich ist.

16. Verfahren zum oxidativen Färben von Keratinfasern und insbesondere menschlichen Keratinfasern, wie den Haaren, **dadurch gekennzeichnet, dass** auf die Fasern mindestens eine Zusammensetzung nach einem der Ansprüche 2 bis 15 aufgebracht und die Farbe durch Luftsauerstoff oder mit Hilfe eines Oxidationsmittels gebildet wird.

17. Verfahren nach Anspruch 16, wobei das Oxidationsmittel unter Wasserstoffperoxid, Harnstoffperoxid, Alkalimetallbromaten, Salzen von Persäuren, Persäuren und Oxidasen ausgewählt ist.

18. Verfahren nach einem der Ansprüche 16 oder 17, wobei das Oxidationsmittel bei der Anwendung mit der Zusammensetzung vermischt wird.

19. Verfahren nach einem der Ansprüche 17 oder 18, wobei das Oxidationsmittel in Form einer oxidierenden Zusammensetzung auf die Fasern aufgebracht wird, die gleichzeitig mit der Zusammensetzung nach einem der Ansprüche 2 bis 15 oder getrennt davon anschließend aufgetragen wird.

20. Vorrichtung mit mehreren Abteilungen oder "Kit" zum Färben mit mehreren Abteilungen, wobei eine Abteilung eine Zusammensetzung nach einem der Ansprüche 2 bis 15 enthält und eine andere Abteilung eine oxidierende Zusammensetzung enthält.

21. Farbiges Produkt, das ausgehend von der Zusammensetzung nach Anspruch 14 durch Reaktion der Verbindung (I) mit der Verbindung (II) erhältlich ist.

22. Produkt nach Anspruch 21, das unter den Produkten der Formeln (IIIa), (IIIb) und (IIIc) und deren Gemischen ausgewählt ist: 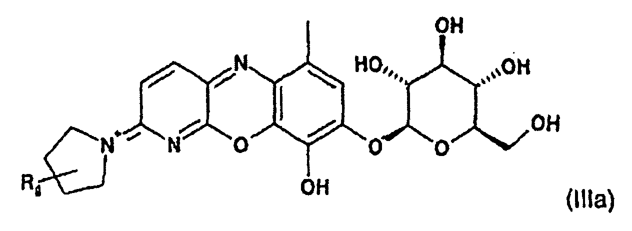 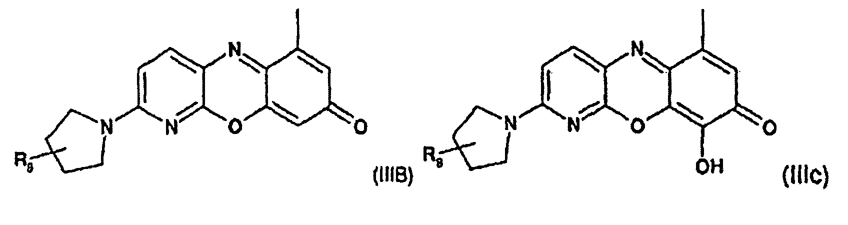 wobei R₈ die in Anspruch 6 angegebene Bedeutung aufweist.

23. Verbindung der folgenden Formel, die aus einem Extrakt von *Myrsine africana* erhältlich ist:

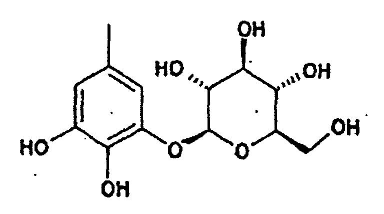

## Claims

1. Use of an extract of *Myrsine africana* as a dye precursor for dyeing keratin fibres by oxidation dyeing.

2. Composition for dyeing keratin fibres by oxidation dyeing, comprising, in a medium that is suitable for dyeing keratin fibres, an extract of *Myrsine africana* and a compound of formula (I), or one of the corresponding cosmetically acceptable salts thereof with an acid or a base: 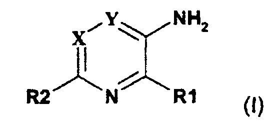 in which
• R₁ represents a radical NR₄R₅ in which R₄ and R₅ represent, independently of each other, a hydrogen; an alkyl radical optionally substituted with one or more hydroxyl, alkoxy, carboxyl, amino, monoalkylamino or dialkylamino groups in which the alkyl radical is optionally substituted with one or more hydroxyl, alkoxy, carboxyl, amino, monoalkylamino or dialkylamino groups; or R₄ and R₅ form, together with the nitrogen atom to which they are attached, a 5- to 8-membered saturated heterocycle, the heterocycle possibly being substituted with one or more halogen atoms, one or more hydroxyl, alkoxy, carboxyl, amino, carboxamido, sulphonamido, monoalkylamino or dialkylamino radicals or C₁-C₆ alkyl radicals optionally substituted with one or more radicals chosen from hydroxyl, amino, carboxyl, monoalkylamino or dialkylamino radicals, preferably C₁-C₂ monoalkylamino or dialkylamino radicals; the heterocycle possibly containing other hetero atoms chosen from O, S, SO₂ and NR", R" representing a hydrogen atom or a C₁-C₄ alkyl radical,
• R₂ represents a radical NR₆R₇ in which R₆ and R₇ represent, independently of each other, a hydrogen; an alkyl radical optionally substituted with one or more hydroxyl, alkoxy, carboxyl, amino, monoalkylamino or dialkylamino groups in which the alkyl radical is optionally substituted with one or more hydroxyl, alkoxy, carboxyl, amino, monoalkylamino or dialkylamino groups; or R₆ and R₇ form, together with the nitrogen atom to which they are attached, a 5- to 8-membered saturated heterocycle, the heterocycle possibly being substituted with one or more halogen atoms, one or more hydroxyl, alkoxy, carboxyl, amino, carboxamido, sulphonamido, monoalkylamino or dialkylamino radicals or C₁-C₆ alkyl radicals optionally substituted with one or more radicals chosen from hydroxyl, amino, carboxyl, monoalkylamino or dialkylamino radicals, preferably C₁-C₂ monoalkylamino or dialkylamino radicals; the heterocycle possibly containing other hetero atoms chosen from O, S, SO₂ and NR", R" representing a hydrogen atom or a C₁-C₄ alkyl radical,
• X-Y represents RC-CR', N-CR with R and R' chosen from a hydrogen atom, an alkyl radical, a hydroxyl radical or an amino radical.

3. Composition according to Claim 2, in which compound (I) corresponds to the following formula: 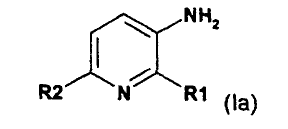 in which R₁ and R₂ are as defined in Claim 2.

4. Composition according to Claim 3, in which R₁ represents NR₄R₅ in which R₄ and R₅ represent hydrogen, an alkyl radical, a hydroxyalkyl radical, or R₄ and R₅ together form a 5- to 7-membered ring, R₂ represents NR₆R₇ in which R₆ and R₇ represent hydrogen, an alkyl radical or a hydroxyalkyl radical, or R₆ and R₇ together form a 5- to 7-membered ring.

5. Composition according to Claim 3 or 4, in which R₄ and R₅ represent a hydrogen or together form a pyrrolidine ring, R₆ and R₇ preferably form a 1-pyrrolidinyl ring, which may be substituted.

6. Composition according to any one of Claims 2 to 5, in which compound (I) corresponds to one of the following formulae 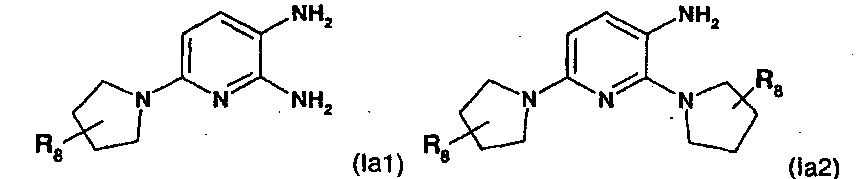 in which R₈ represents a hydrogen atom, a halogen atom, a hydroxyl, alkoxy, carboxyl, amino, carboxamido, sulphonamido, monoalkylamino or dialkylamino radical or a C₁-C₆ alkyl radical optionally substituted with one or more hydroxyl, amino, carboxyl, monoalkylamino or dialkylamino radicals, preferably C₁-C₂ monoalkylamino or dialkylamino radicals.

7. Composition according to Claim 2, in which compound (I) corresponds to the following formula: 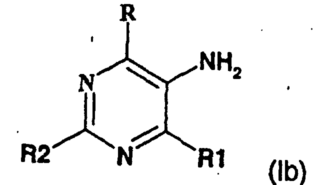 in which R, R₁ and R₂ are as defined in Claim 2.

8. Composition according to Claim 7, in which compound (Ib) is such that R is an amino or hydroxyl radical.

9. Composition according to Claim 8, in which compound (Ib) is such that R, R₁ and R₂ represent NH₂.

10. Composition according to any one of Claims 2 to 9, comprising a compound of formula (II) below

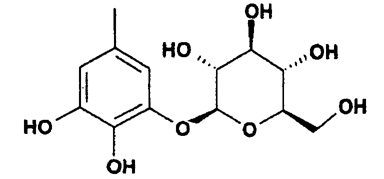

11. Composition according to any one of Claims 2 to 10, in which the amount of extract of *Myrsine africana* is between 0.1% and 10% by weight.

12. Composition according to any one of Claims 2 to 11, also comprising one or more oxidation bases chosen from para-phenylenediamines, bis(phenyl)alkylenediamines, para-aminophenols, ortho-aminophenols and heterocyclic bases, and the addition salts thereof.

13. Composition according to any one of Claims 2 to 12, also comprising one or more couplers chosen from meta-phenylenediamines, meta-aminophenols, meta-diphenols, naphthalene-based couplers and heterocyclic couplers, and also the addition salts thereof.

14. Composition according to any one of Claims 2 to 13, also comprising a direct dye.

15. Composition for the oxidation dyeing of keratin fibres, comprising, in a medium that is suitable for dyeing keratin fibres, a dye precursor of formula (I) as defined in any one of Claims 2 to 9 and 3-(β-D-glucopyranosyloxy)-4,5-dihydroxytoluene which may be obtained from an aqueous extract of *Myrsine africana*.

16. Process for the oxidation dyeing of keratin fibres, and in particular of human keratin fibres such as the hair, **characterized in that** at least one composition as defined in any one of Claims 2 to 15 is applied to the fibres, and the colour is revealed with atmospheric oxygen or using an oxidizing agent.

17. Process according to Claim 16, in which the oxidizing agent is chosen from hydrogen peroxide, urea peroxide, alkali metal bromates, persalts, peracids and oxidase enzymes.

18. Process according to either of Claims 16 and 17, in which the oxidizing agent is mixed with the composition at the time of use.

19. Process according to either of Claims 17 and 18, in which the oxidizing agent is applied to the fibres in the form of an oxidizing composition simultaneously with or sequentially to the composition as defined in any one of Claims 2 to 15.

20. Multi-compartment device or multi-compartment dyeing "kit", a first compartment of which contains a composition as defined in any one of Claims 2 to 15, and a second compartment of which contains an oxidizing composition.

21. Coloured product that may be obtained using the composition as defined in Claim 14 by reacting compound (I) with compound (II).

22. Product according to Claim 21, chosen from the products of formulae (IIIa), (IIIb) and (IIIc) and mixtures thereof: 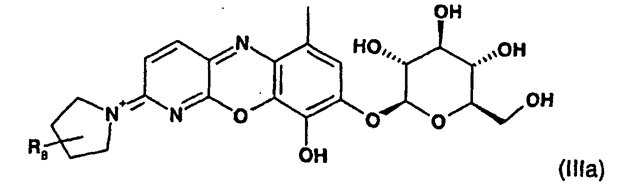 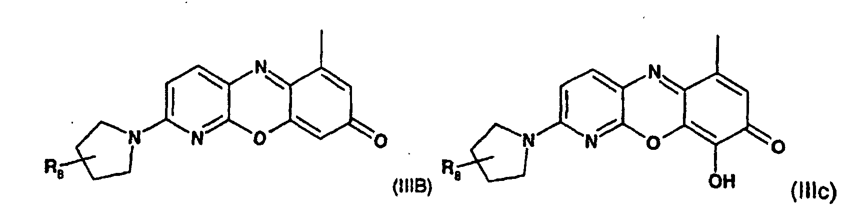 in which R₈ is as defined in Claim 6.

23. Compound having the formula below, which may be obtained from an extract of *Myrsine africana*:
